(19) Europäisches Patentamt
European Patent Office
Office européen des brevets

(11) Numéro de publication : 0 272 191 B1

# (12) FASCICULE DE BREVET EUROPEEN

(45) Date de publication du fascicule du brevet : 27.02.91 Bulletin 91/09

(51) Int. Cl.$^5$ : C07C 69/593, C07C 67/36

(21) Numéro de dépôt : 87420306.0

(22) Date de dépôt : 10.11.87

## (54) Procédé de préparation de diesters de l'acide hexenedioique.

(30) Priorité : 14.11.86 FR 8616046

(43) Date de publication de la demande : 22.06.88 Bulletin 88/25

(45) Mention de la délivrance du brevet : 27.02.91 Bulletin 91/09

(84) Etats contractants désignés : BE DE ES FR GB IT NL

(56) Documents cités :
FR-A- 1 419 758
GB-A- 987 274

(73) Titulaire : RHONE-POULENC CHIMIE
25, quai Paul Doumer
F-92408 Courbevoie Cédex (FR)

(72) Inventeur : Denis, Philippe
16, Allée des Troenes
F-69150 Decines (FR)
Inventeur : Frances, Jean-Marc
9, Avenue Condorcet
F-69100 Villeurbanne (FR)

(74) Mandataire : Varnière-Grange, Monique et al
RHONE-POULENC INTERSERVICES Service Brevets Chimie Centre de Recherches des Carrières B.P. 62
F-69192 Saint-Fons Cédex (FR)



Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention a pour objet un procédé de préparation de diesters de l'acide hexènedioïque-1,6. Ces diesters peuvent être hydrogénés en diesters de l'acide adipique correspondants ou adipates qui peuvent alors être hydrolysés pour former l'acide adipique. L'acide adipique, l'une des matières premières du nylon 66 est produit avec de forts tonnages et de ce seul fait, toute nouvelle voie d'accès à ce diacide et/ou à ses dérivés présente un intérêt de principe immédiatement perceptible.

La présente invention a plus spécifiquement pour objet un procédé de préparation de diesters de l'acide hexène-3 dioïque par réaction du monoxyde de carbone et d'un alcool sur un dichlorobutène en présence d'un catalyseur à base de palladium.

Il est connu (cf. Journal of the American Chemical Society, 86, 1964 p. 4 351) que l'on peut obtenir le vinylacétate d'éthyle par réaction, à 120°C sous 130 atm environ, du monoxyde de carbone, de l'éthanol et du chlorure d'allyle, en présence de chlorure de palladium (II).

Il est bien connu également [cf. Journal of Organometallic Chemistry, 188, 1980, p. 229) que l'on peut obtenir le vinylacétate de méthyle par réaction, à 80°C sous 200 atm, du monoxyde de carbone, du méthanol et du chlorure d'allyle, en présence d'un catalyseur à base de chlorure de bis(triphénylphosphine) palladium (II) et de chlorure d'étain (II) dans la méthylisobutylcétone ou le benzène.

Toutefois une simple transposition de ces techniques à un substrat tel le dichloro-1,4 butène-2 ou le dichloro-3,4 butène-1 ne conduit pas aux diesters recherchés.

Par ailleurs dans la demande de brevet japonais publiée sous le n° 68/23 929 il est décrit un procédé dans lequel du diéthoxy-1,4 butène-2 est mis à réagir avec du monoxyde de carbone, de l'éthanol en présence d'acétylacétonate de palladium (II) et d'acide chlorhydrique, à une température de 160°C, la pression initiale du monoxyde de carbone étant de 150 Kg/cm$^2$, procédé par lequel on obtient notamment l'ester diéthylique de l'acide héxène-3 dioïque. Toutefois la sélectivité en hexène-3 dioate reste faible malgré des conditions relativement dures de température, de pression et de durée de réaction (cf. également l'étude détaillée faite par les mêmes auteurs dans Tetrahedron Vol. 25, p. 4 189-4 190, 1969).

Dans le cadre de la recherche de nouvelle voie d'accès à l'acide adipique, il s'avérait donc nécessaire de proposer une manière efficace pour accéder aux diesters linéaires l'acide hexène-3 dioïque appelés plus brièvement hexènedioates-1,6 qui après hydrogénation conduisent à l'adipate correspondant. Il s'avérait également important de proposer des moyens efficaces pour accéder aux hexènedioates-1,6 à partir de dichlorobutène et plus particulièrement à partir du dichloro-1,4 butène-2, du dichloro-3,4 butène-1 ou de leurs mélanges en toutes proportions.

Il est par ailleurs connu que la chloration en phase gazeuse du butadiène, par exemple en présence de catalyseurs à base de palladium et de cuivre conduit facilement à la formation sélective d'un mélange de dichloro-1,4 butène-2 et de dichloro-1,2 butène-3 (cf. le brevet des Etats Unis d'Amérique n° 3 823 096).

Il a maintenant été trouvé et, c'est ce qui constitue l'objet essentiel de la présente invention, qu'il est possible d'accéder efficacement et sélectivement aux hexènedioates-1,6 à partir du monoxyde de carbone, d'un alcool et d'au moins un dichlorobutène.

La présente invention a donc pour objet un procédé de préparation d'hexènedioates-1,6 par réaction du monoxyde de carbone, d'un alcool et d'au moins un dichlorobutène en présence d'une quantité catalytiquement efficace de palladium ou d'un composé du palladium, la réaction étant assistée par une amine tertiaire dont la quantité n'excède pas 2 équivalents par rapport au dichlorobutène.

Les produits visés dans le cadre de la mise en oeuvre du présent procédé peuvent être représentés par la formule (I) ci-après :

$$RO-\underset{\underset{O}{\|}}{C}-CH_2-CH=CH-CH_2-\underset{\underset{O}{\|}}{C}-OR \qquad (I)$$

dans laquelle R représente un radical alkyle linéaire ou ramifié comportant au maximum 12 atomes de carbone dans la chaîne principale, pouvant comporter un ou deux substituants alkyles ayant au maximum 4 atomes de carbone, ou un ou deux groupes hydroxyles, un radical cycloalkyle ayant de 5 à 7 atomes de carbone, un radical aralkyle ayant de 7 à 12 atomes de carbone ou un radical phényle.

Le radical R dérive de l'alcool ROH, l'un des réactifs nécessaires à la mixe en oeuvre de la présente invention.

Par dichlorobutène on entend essentiellement le dichloro-1, 4 butène-2, le dichloro-3,4 butène-1 et leurs mélanges.

Comme indiqué ci-avant la réaction est assistée par une amine tertiaire. L'amine tertiaire répond à l'une

quelconque des formules (II) et (III) ci-après.

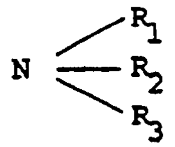

(II)

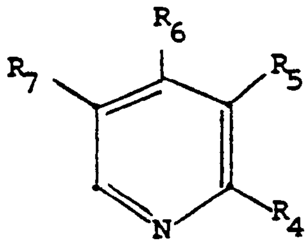

(III)

dans lesquelles :

– $R_1$, $R_2$ et $R_3$ sont identiques ou différents et répresentent :

• un radical alkyle linéaire ou ramifié contenant de 1 à 16 atomes de carbone éventuellement substitué par un groupe phényle ;

• un radical alcényle linéaire ou ramifié contenant de 2 à 12 atomes de carbone, de préférence de 4 à 8 atomes de carbone ;

• un radical aryle contenant de 6 à 10 atomes de carbone éventuellement substitué par un ou deux radicaux alkyles contenant de 1 à 4 atomes de carbone ;

• deux desdits radicaux $R_1$ à $R_3$ pouvant former ensemble un radical alkylène, alcénylène ou alcadiènylène, linéaire ou ramifié et contenant de 3 à 6 atomes de carbone ou un groupement formé de deux radicaux alkylènes, identiques ou différents, comportant de 1 à 3 atomes de carbone et reliés entre eux par un atome d'oxygène ou de soufre ;

• les trois radicaux $R_1$ à $R_3$ pouvant être reliés entre eux pour former avec l'atome d'azote un composé à deux cycles ortho-condensés, l'atome d'azote étant commun aux deux cycles ;

• les trois radicaux $R_1$ à $R_3$ pouvant en outre définir avec l'atome d'azote un hétérocycle comportant une double liaison carbone-azote, comportant le cas échéant une double liaison carbone-carbone éventuellement commune à l'hétérocycle et à un noyau benzénique.

– $R_4$, $R_5$, $R_6$ et $R_7$ sont identiques ou différents et représentent :

• un atome d'hydrogène ;

• un radical alkyle linéaire ou ramifié contenant de 1 à 4 atomes de carbone ;

• deux des radicaux $R_4$ à $R_7$, portés par des atomes de carbones adjacents du cycle, pouvant former ensemble un radical akylène, alcénylène ou alcadiènylène, contenant 4 atomes de carbone ou un groupement formé de deux radicaux alkylènes, identiques ou différents, comportant de 1 à 3 atomes de carbone et reliés entre eux par un atome d'oxygène ou de soufre ;

A titre d'exemples d'amines tertiaires utilisables dans le cadre du présent procédé on peut citer :

la triméthylamine
la triéthylamine
la tri(n-butyl) amine
la di(isopropyl)éthylamine
la benzyldiméthylamine
la méthyldiphénylamine
la diméthylphénylamine
l'éthylméthylphénylamine
la méthyl(n-propyl)phénylamine
l'éthylphénylpropylamine
la diéthylphénylamine

la di(n-propyl)phénylamine
la N-éthylmorpholine,
la N-méthylpyrrolidine,
l'indolizine,
le 2H-pyrrole
le 3H-indole
la pyridine
la picoline -2 (ou 4)
la lutidine -3,5
l'isoquinoleïne
la quinoléine

Une première classe d'amines tertiaires convenant à la mise en oeuvre de la présente invention répond à la formule II ci-avant.

Parmi celles-ci on préfère celles correspondant aux cas où l'un au moins des radicaux $R_1$ à $R_3$ représente un radical alkyle ou alcényle linéaire non substitué ou aux cas où lorsque l'un des radicaux $R_1$ à $R_3$ représente un radical aryle, les deux autres qui peuvent être identiques ou différents, représentent des radicaux alkyles linéaires et non substitués. Les radicaux $R_1$ à $R_3$ sont avantageusement identiques et choisis parmi les radicaux alkyles linéaires contenant au maximum 4 atomes de carbone. On préconise plus particulièrement l'emploi de la triéthylamine dans cette première classe d'amines tertiaires.

Une deuxième classe d'amines tertiaires convenant à la mise en oeuvre du présent procédé répond à la formule III ci-avant.

Parmi celles-ci on préférera celles correspondant aux cas où moins deux des radicaux $R_4$ à $R_7$ représentent l'hydrogène et ou les deux autres radicaux identiques ou différents représentent un radical alkyle linéaire comportant au maximum 4 atomes de carbone, ces deux radicaux lorsqu'ils sont portés par des atomes de carbone adjacents du cycle pouvant former ensemble un radical alkylène, alcénylène ou alcadiénylène contenant 4 atomes de carbone ou un groupement formé de deux radicaux éthylène reliés entre eux par un atome d'oxygène.

On préconise plus particulièrement l'emploi de la pyridine dans cette seconde classe d'amines tertiaires.

Comme indiqué en tête du présent mémoire la réaction est assistée par une amine tertiaire en ce sens que cette amine est un composant indispensable à la bonne marche de la réaction. La demanderesse sans limiter en cela la portée de son invention ou être liée par les explications fournies ci-après, se propose de représenter la réaction en cause par les équations réactionnelles 1 (a,b) et 2 écrites dans l'hypothèse où l'on part du dichloro-1,4 butène-2, du méthanol et de la triéthylamine :

<u>Equation 1</u> (a):

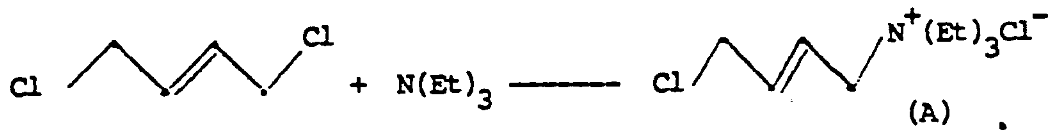

(A) .

Equation 1 (b):

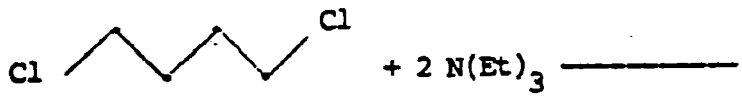

(B)

(B) :

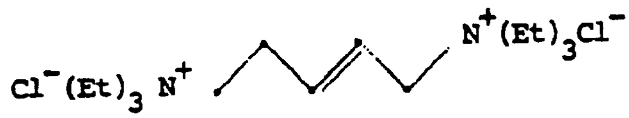

Equation 2 :

A et/ou B

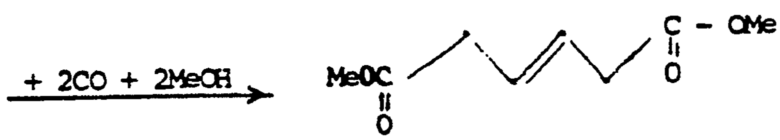


$+[HCl + HN^+(Et)_3Cl^-]$ et/ou $2[HN^+(Et)_3Cl^-]$

La Demanderesse, dans le cadre des travaux de recherche qui ont abouti à la présente invention a d'une part, constaté que le chlorure d'ammonium quaternaire formé dans l'équation 1 (a ou b) est le même lorsqu'on part du dichloro-3,4 butène-1 et, d'autre part, mis en évidence le rôle déterminant de ce chlorure d'ammonium quaternaire dans la réaction d'alcoxycarbonylation à proprement parler.

La Demanderesse a également mis en évidence l'absence de la réaction souhaitée lorsque l'amine tertiaire représente plus de deux équivalents par rapport au dichlorobutène engagé.

Si l'effet de l'amine tertiaire se fait sentir de manière significative dès qu'elle est introduite à raison 0,05 mole par mole de dichlorobutène engagé, pour une bonne mise en oeuvre de l'invention, la quantité d'amine tertiaire sera au moins équimolaire à plus ou mois 20% près et de préférence, à plus ou moins 10% près, du dichlorobutène engagé.

Comme les équations ci-avant l'indiquent le dichlorobutène est d'abord au moins pour partie transformé en chlorure d'ammonium quaternaire ; ce chlorure d'ammonium quaternaire qui résulte de la fixation d'une (à deux) mole(s) d'amine tertiaire par mole de dichlorobutène serait alors le substrat de la réaction par laquelle deux fonctions RO(CO)-, R ayant la signification donnée en tête du présent mémoire, se fixent sur la chaîne carbonée dérivant du dichlorobutène. Ces deux réactions peuvent être réalisées en un seul stade opératoire ou bien être l'objet de deux opérations distinctes.

Dans le mode de réalisation en un seul stade on met à réagir dans un réacteur unique, le dichlorobutène, l'alcool, l'amine tertiaire et le monoxyde de carbone en présence du catalyseur. Le chlorure d'ammonium quaternaire en cause est produit in-situ dans les conditions de la réaction "d'alcoxycarbonylation" qui seront détaillées ultérieurement.

Dans le mode de réalisation en deux stades on prépare, dans un premier temps, le chlorure d'ammonium quaternaire en cause par mise en réaction du dichlorobutène et de l'amine tertaire en proportion sensiblement équimolaire. Après une durée de réaction généralement comprise entre 30 et 60 minutes à une température de l'ordre de 20 à 50°C le sel d'ammonium précipite, il est alors récupéré par simple filtration et le cas échéant purifié par simple lavage à l'éther diéthylique, par exemple. Il est tout à fait envisageable de recourir à des solvants ou diluants au cours de ce stade de préparation.

Dans un second stade qui peut-être réalisé dans le même appareillage que le premier stade (phase de quaternisation), sous réserve toutefois que celui-ci soit résistant à la pression, on met à réagir le chlorure d'ammonium quaternaire produit au premier stade et le cas échéant, purifié par tout moyen approprié, et eventuellement une quantité supplémentaire d'amine tertiaire sous réserve toutefois que la quantité totale engagée dans les deux stades, n'excède pas la quantité maximale prescrite en tête du présent mémoire, l'alcool et le monoxyde de carbone en présence du catalyseur au palladium.

Les conditions de la réaction "d'alcoxycarbonylation" seront détaillées ultérieurement.

Que l'on choisisse d'opérer en un ou deux stades, la réaction par laquelle deux fonctions RO(CO)-, R ayant la signification précédente, se fixent sur la chaîne carbonée dérivant du dichlorobutène, réaction,

appelée pour simplifier l'exposé "alcoxycarbonylation", est conduite en phase liquide en présence d'une quantité catalytiquement efficace de palladium ou d'un composé du palladium.

On opère avantageusement avec du palladium au degré d'oxydation (II) ou zéro (0) dont la majeure partie est soluble dans le milieu et dans les conditions de réaction.

A titre d'exemple de composés du palladium (II) ou (0) susceptibles de convenir à la mise en oeuvre du présent procédé on peut citer :

- les sels d'acides organiques tels l'acétate de palladium (II), le formiate de palladium (II), le propionate de palladium (II), l'octanoate de palladium (II), l'éthylhexanoate de palladium (II) ;
- les sels d'acides minéraux tels le nitrate et le chlorure de palladium (II) ;
- les complexes $\pi$-allyliques de palladium (II) tel le chloro ($\pi$-allyl)palladium ;
- l'acétylacétonate de palladium (II).
- les complexes du palladium et d'une trialkyl- ou triarylphosphine tel le tétrakis(triphénylphosphine)palladium.
- Les composés du palladium (0) et de la dibenzylidèneacétone (dba) $Pd(dba)_2$, $Pd_2(dba)_3$, $Pd(dba)_3$ et leurs mélanges.

Conviennent plus particulièrement le bis(dibenzylidèneacétone) palladium et le chlorure de palladium.

La quantité de palladium à mettre en oeuvre dans le cadre du procédé selon l'invention peut varier dans de larges limites. La quantité sera en pratique choisie de telle sorte que le procédé soit économiquement intéressant.

La bonne activité des catalyseurs au palladium permet l'emploi desdits catalyseurs en quantité très faible (correspondant à un rapport molaire palladium/dichlorobutène éventuellement quaternisé de l'ordre de 0,01 : 100 ; l'emploi d'une quantité élevée de catalyseur (correspondant à un rapport molaire palladium/dichlorobutène éventuellement quaternisé de l'ordre de 10 : 100 n'est pas nuisible. Dans la mesure où le but recherché est de réaliser une opération d'alcoxycarbonylation suffisamment rapide et sélective, sans consentir une dépense exhorbitante pour le palladium, un rapport palladium/dichlorobutène éventuellement quaternisé compris entre 0,5 : 100 et 5 : 100 est généralement préférable.

La réaction d'alcoxycarbonylation nécessite la mise en oeuvre d'un alcool de formule ROH, R ayant la signification donnée précédemment.

A titre d'exemples d'alcools utilisables dans le cadre du présent procédé on peut citer le méthanol, l'éthanol, l'isopropanol, le n-propanol, le tertio butanol, le n-hexanol, le cyclohexanol, l'éthyl-2 hexanol-1, le dodécanol-1, l'éthylèneglycol, l'hexanediol-1,6, l'alcool benzylique, l'alcool phényléthylique et le phénol.

On utilise de préférence un alcanol ayant au maximum 4 atomes de carbone ; le méthanol et l'éthanol conviennent bien à la mise en oeuvre du présent procédé.

La réaction d'alcoxycarbonylation met en cause par sa stoechiométrie propre 2 moles d'alcool par mole de substrat, en entendant ici par substrat le dichlorobutène ou le chlorure d'ammonium quaternaire formé selon le principe de l'équation réactionnelle (1). Néanmoins la quantité d'alcool peut varier dans de larges limites.

Ainsi, bien qu'il soit possible d'utiliser de 0,5 à 10 fois la quantité d'alcool stoechiométriquement nécessaire, il est préférable afin d'avoir une conversion maximale du substrat tout en évitant de trop diluer le milieu par de l'alcool, de réaliser le procédé avec un rapport molaire alcool/substrat compris entre environ 0,8 et 5.

La réaction d'alcoxycarbonylation requiert la présence de monoxyde de carbone. On met en oeuvre de préférence, du monoxyde de carbone sous forme essentiellement pure, tel qu'il se présente dans le commerce. Toutefois la présence d'impuretés telles que du dioxyde de carbone, de l'oxygène, de l'hydrogène, du méthane et de l'azote peut être tolérée.

La réaction d'alcoxycarbonylation est généralement conduite à une température supérieure ou égale à 60°C, il ne paraît pas souhaitable de dépasser une température de 160°C pour limiter les réactions de dégradations des produits de départ.

De bons résultats sont obtenus lorsque la température est comprise entre 70 et 110°C.

On opère en phase liquide sous une pression généralement supérieure à 50 bar pour obtenir une vitesse de conversion appréciable. Il n'est pas utile d'atteindre 700 bar. Pour une bonne mis en oeuvre de l'invention, une pression totale de 80 à 200 bar est préconisée.

Comme l'équation 2 en tête du présent mémoire le fait apparaître, il y a présence simultanée du méthanol et d'acide chlorhydrique ce qui peut générer de l'eau. Cette présence d'eau étant nuisible à la sélectivité en produits visés, il est avantageux de conduire la réaction d'alcoxycarbonylation en présence d'un agent deshydratant, par exemple, le diméthoxy-2,2 propane.

Lorsqu'on opère avec une quantité d'amine tertiaire voisine de la quantité maximale indiquée, l'acide chlorhydrique est pour tout ou partie bloqué sous forme du chlorhydrate correspondant à l'amine utilisée.

Il s'avère alors superflu d'introduire un agent deshydratant.

La présence de solvant ou diluant n'est pas nécessaire à la mise en oeuvre du procédé selon l'invention. Toutefois on peut faire usage de solvants ou diluants, par exemple, d'hydrocarbures aromatiques chlorés ou non, d'alcanes chlorés et de diméthylformamide. On aura alors recours de préférence à des solvants solubilisant les produits de départ ou intermédiaires tels le diméthylformamide ou le dichlorométhane.

En fin d'opération, l'hexènedioate-1,6 obtenu est séparé des autres constituants du milieu réactionnel par tout moyen approprié, par exemple par distillation.

Le présent procédé convient particulièrement bien à la préparation de l'hexène-3 dioate de méthyle (ou d'éthyle).

Les exemples ci-après illustrent l'invention sans toutefois en limiter le domaine ou l'esprit.

Les conventions suivantes y sont utilisées.

– RR désigne le rapport du nombre de moles de produits formés au nombre de moles de produits de départ, qu'il s'agisse de dichlorobutène ou de dichlorobutène quaternisé.

EXEMPLES 1 à 9 ; essai temoin (a) :

Une première serie d'essais est réalisée selon le mode opératoire suivant.

Dans un autoclave en acier inoxydable HASTELLOY B2$^R$ de 125 $cm^3$ préalablement purgé à l'argon, on introduit (sauf mention contraire) :

– 6,25 g (50 mmol) de dichloro-1,4 butène-2,

– 15 g (469 mmol) de méthanol,

– 1,25 milliatome-gramme de palladium sous forme de $PdCl_2$ [ou de $Pd(dba)_2$ dans les exemples marqués d'une croix],

– le cas échéant une amine tertiaire dont la nature et la quantité seront indiquées ci-après.

L'autoclave est fermé hermétiquement, placé dans un four agité et raccordé à l'alimentation de gaz sous pression. On admet à froid, 50 bar de monoxyde de carbone et on chauffe à 95°C. Lorsque cette température est atteinte, on régule la pression à 120 bar. Après une durée de réaction (t) l'autoclave est refroidi et ramené à la pression atmosphérique. La solution réactionnelle est alors filtrée puis analysé par chromatographie en phase gazeuse. Les produits formés sont essentiellement :

– l'hexène-3 dioate de méthyle (H3D)

– les penténoates de méthyle (P3)

– le pentadiènoate de méthyle (PD)

Les conditions particulières ainsi que les résultats obtenus sont rassemblés dans le tableau (I) ci-après:

TABLEAU I

| N° Exemple | Amine tertiaire | | Temps | RR (en %) | | |
|---|---|---|---|---|---|---|
| | Nature | mmol | | H3D | P3 | PD |
| 1 | triéthylamine | 50 | 1h 15mn | 8,5 | 3,5 | 0,5 |
| 2(x) | pyridine | 33 | 1h 50mn | 8,5 | ND | 0,6 |
| 3(x) | triéthylamine | 50 | 1h 20mn | 7,5 | 25 | 2,5 |
| 4(xx) | triéthylamine | 50 | 1h 30mn | 7,5 | 28,5 | 1,5 |
| 5 | N-éthylmorpholine | 50 | 1h 50mn | 7 | 28 | 1,2 |
| 6 | tributylamine | 50 | 3h | 4,5 | 47 | 0 |
| 7 | lutidine-3,5 | 50 | 1h 50mn | 15,5 | 12 | 11 |
| 8 | N-méthylpyrrolidine | 50 | 50mn | 8,5 | 8,5 | 18 |
| 9 | N-méthylpyrrolidine | 100 | 2h | 11,1 | 0,5 | 27,5 |
| a(xxx) | néant | 0 | 1h 30 | 0 | - | - |

(x) = $Pd(dba)_2$
(xx) = 5m.At-g de palladium,
(xxx) = en fin d'essai on dose :
- 2,8 mmol de pentanoate et méthylbutanoate de méthyle,
- 25 mmol de penténoates et méthylbuténoates de méthyle,
- 0 mmol de dichloro-1,4 butène-2,

L'essai (a) montre qu'en l'absence d'amine tertiaire la réaction recherchée n'a pas lieu.

Exemples 10 à 15 :

Une seconde série d'essais est réalisée dans l'appareillage et selon un mode opératoire analogue à ceux décrits pour les exemples précédents, à ceci près qui l'admission de la pression de monoxyde de carbone de 50 bar est précédée par une opération de chauffage à 50°C pendant un temps $t_1$, la durée de la réaction en présence du monoxyde de carbone étant désignée par $t_2$. La charge est conforme à celle décrite précédemment.

Les conditions particulières et les résultats obtenus sont indiqués dans le tableau (II) ci-après :

TABLEAU II

| N° Exemple | Amine (mmol) | | $t_1$ | $t_2$ | RR (en %) H3D | P3 | PD |
|---|---|---|---|---|---|---|---|
| 10 | triéthylamine | 50 | 1h 30mn | 1h 30mn | 21 | 12,5 | 7,2 |
| 11 | ,, | 70 | 2h | 1h 30mn | 32,5 | 5,2 | 8 |
| 12 | ,, | 90 | 2h 00 | 2h 45mn | 36,5 | 3,1 | 13 |
| 13 | ,, | 90 | 4h | 10h | 35 | ND | 13 |
| 14 | pyridine | 70 | 1h 30mn | 3h | 44 | 6,3 | 2,5 |
| 15 | ,, | 70 | 45mn | 3h | 45 | 6,1 | 4,6 |

Exemples 16 à 25 :

Une troisième série d'essais est réalisée dans l'appareillage et selon un mode opératoire analogue à ceux décrits pour les exemples 1 à 9, à ceci près que le dichloro-1,4 butène est remplacé dans la charge par une quantité équivalente de chlorure de (chloro-4 butène-2 yle) triéthylammonium. Certains essais ont portés sur une charge dans laquelle on a remplacé 11 g de méthanol par un solvant dont la nature et la quantité sont indiquées ci-après.

Les conditions particulières et les résultats obtenus sont indiqués dans le tableau (III) ci-après :

TABLEAU III

| N° Ex. | Amine Nature | Amine mmol | Solvant Nature | Solvant g | temps | RR (en %) H3D | RR (en %) P3 | RR (en %) PD |
|---|---|---|---|---|---|---|---|---|
| 16 | - | 0 | - | 0 | 1h 20mn | 52,5 | 6 | 6,5 |
| 17 | triéthylamine | 40 | - | 0 | 1h 50mn | 27,5 | 2,5 | 17,5 |
| 18 | pyridine | 40 | - | 0 | 2h 15mn | 63,3 | 2 | 8 |
| 19 | N-méthylpyr-rolidine | 40 | - | 0 | 2h 10mn | 29,3 | 2,8 | 22 |
| 20 | tributylamine | 40 | - | 0 | 2h 10mn | 35,7 | 3,4 | 22 |
| 21 | pyridine | 45 | | 0 | 2h 20mn | 63,5 | 1,75 | 12,8 |
| 22 | - | 0 | $CH_2Cl_2$ | 21 | 3h 50mn | 11,7 | 16,2 | 0 |
| 23 | - | 0 | DMF | 15 | 2h 30mn | 37,1 | 8,0 | 0 |
| 24 | - | 0 | $CH_3CN$ | 12,5 | 3h 30mn | 28,2 | 16 | 0 |
| 25 | - | 0 | MCB | 21 | 3h 20mn | 14,7 | 21,5 | 0 |

MCB = Monochlorobenzène ; DMF = Diméthylformamide.

Exemples 26 à 34 :

Une quatrième série d'essais est réalisée dans l'appareillage décrit ci-avant sur une charge renfermant:
- 50 mmol de chlorure de (chloro-4 butène-2 yle)triéthylammonium
- 10 g de méthanol,
- 1,25 m at-g de palladium sous forme de $PdCl_2$ [sauf dans l'exemple 29 où il est chargé sous forme de Pd $(dba)_2$]
- 10 g diméthoxypropane (sauf dans l'exemple 26 où il n'y en a que 5 g). Le mode opératoire utilisé est soit celui décrit pour la première série d'essais (préchauffage conduit en présence de monoxyde de carbone) et indiqué par (I) ci-après soit celui décrit pour la seconde série d'essais (l'admission de la pression de 50 bar de monoxyde de carbone est précédée par un préchauffage à 95°C) et indiqué par (II) ci-après

Les conditions particulières et les résultats obtenus sont indiqués dans le tableau (IV) ci-après :

TABLEAU IV

| N° Exemple | | T°C | P Bars | t | RR (en %) H3D | P3 | PD |
|---|---|---|---|---|---|---|---|
| 26 (x) | (I) | 95 | 120 | 1h 40mn | 65,6 | 10,4 | 3,5 |
| 27 | ,, | ,, | ,, | 2h 20mn | 72 | 7,1 | 6,15 |
| 28 | (II) | ,, | ,, | 2h | 76,5 | 8,5 | 8,5 |
| 29 (xx) | ,, | ,, | ,, | 2h 30mn | 71,6 | 9,0 | 6,2 |
| 30 | ,, | ,, | 80 | 3h 30mn | 62 | 5,9 | 9,6 |
| 31 | ,, | ,, | 160 | 1h 30mn | 82 | 9,0 | 6,6 |
| 32 | ,, | ,, | 200 | 1h 40mn | 76,7 | 7,0 | 7,0 |
| 33(xxx) | ,, | 80 | 160 | 4h | 86 | 9,0 | 6,5 |
| 34 | (I) | 110 | 160 | 1h 30mn | 70,5 | 7,8 | 5,5 |

(x) = 5 g de diméthoxypropane
(xx) = $Pd(dba)_2$
(xxx) = T : 80°C; puis 95°C.

Exemples 36 à 37 :

Une cinquième série d'essais est réalisée dans l'appareillage et selon un mode opératoire décrit pour la première série d'essais, la charge renfermant :

- 50 mmol de chlorure de bis(triéthylammonium)-1,4 butène-2,
- du méthanol,
- le cas échéant du diméthoxypropane (DMP)
- 1,25 mAt-g de palladium sous forme de $PdCl_2$ (exemple 35 et 37) ou de $Pd(dba)_2$ (exemple 36).

Les conditions particulières et les résultats obtenus à 95°C sous 120 bar de monoxyde de carbone sont indiqués dans le tableau (V) ci-après :

TABLEAU V

| N° Exemple | MeOH (g) | Divers | t | RR (en %) H3D | P3 | PD |
|---|---|---|---|---|---|---|
| 35 | 10 | Pd(2) | 2h 20mn | 51,0 | 1,5 | 11 |
| 36 | 15 | Pd(o) | 1h 40mn | 42,2 | 2,6 | 1,5 |
| 37 | 12 | Pd(2) + 8,5g DMP | 3h 40mn | 65,0 | 8,5 | 6,7 |

**Revendications**

1. Procédé de préparation d'hexènedioates-1,6 par réaction du monoxyde de carbone, d'un alcool, et d'au moins un dichlorobutène en présence d'une quantité catalytiquement efficace de palladium ou d'un composé du palladium, la réaction étant assistée par une amine tertiaire dont la quantité n'excède pas 2 équivalents par rapport au dichlorobutène.

2. Procédé de préparation d'hexènedioates-1,6 par réaction du monoxyde de carbone, d'un alcool et d'au moins un chlorure d'ammonium quaternaire, ledit chlorure résultant de la quaternisation d'une amine tertiaire par un dichlorobutène, en présence d'une quantité catalytiquement efficace de palladium ou d'un composé du palladium.

3. Procédé de préparation d'hexènedioates-1,6 comprenant la préparation dans un premier stade d'un chlorure d'ammonium quaternaire, par quaternisation d'une amine tertiaire au moyen d'un dichlorobutène, suivie le cas échéant de la séparation dudit chlorure d'ammonium quaternaire du milieu de réaction, et dans un second stade la mise en réaction dudit chlorure avec du monoxyde de carbone et d'un alcool en présence d'une quantité catalytique efficace de palladium ou d'un composé du palladium et, le cas échéant d'une amine tertiaire, dont la quantité totale utilisée dans les deux stades n'excède pas 2 équivalents par rapport au dichlorobutène.

4. Procédé selon l'une quelconque des revendications précédentes, caractérisé en ce que l'amine tertiaire est engagée à raison de 1 équivalent plus ou moins 20% par rapport au dichlorobutène.

5. Procédé selon l'une quelconque des revendications précédentes, caractérisé en ce que l'amine tertiaire est engagée à raison de 1 équivalent plus ou moins 10% par rapport au dichlorobutène.

6. Procédé selon l'une quelconque des revendications précédentes caractérisé en ce que la réaction du monoxyde de carbone, d'un alcool et du dichlorobutène le cas échéant quaternisé, est conduite en phase liquide.

7. Procédé selon l'une quelconque des revendications précédentes caractérisé en ce que l'amine tertiaire répond à l'une quelconque des formules (II) et (III) ci-après.

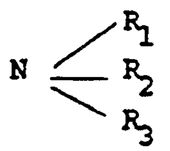

(II)

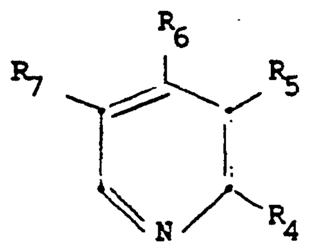

(III)

dans lesquelles :

– $R_1$, $R_2$ et $R_3$ sont identiques ou différents et représentent :

• un radical alkyle linéaire ou ramifié contenant de 1 à 16 atomes de carbone éventuellement substitué par un groupe phényle ;

• un radical alcényle linéaire ou ramifié contenant de 2 à 12 atomes de carbone, de préférence de 4 à 8 atomes de carbone ;

• un radical aryle contenant de 6 à 10 atomes de carbone éventuellement substitué par un ou deux radicaux alkyles contenant de 1 à 4 atomes de carbone ;

• deux desdits radicaux $R_1$ à $R_3$ pouvant former ensemble un radical alkylène, alcénylène ou alcadiènylène, linéaire ou ramifié et contenant de 3 à 6 atomes de carbone ou un groupement formé de deux radicaux alkylènes, identiques ou différents, comportant de 1 à 3 atomes de carbone et reliés entre eux par un atome d'oxygène ou de soufre ;

• les trois radicaux $R_1$ à $R_3$ pouvant être reliés entre eux pour former avec l'atome d'azote un composé à deux cycles ortho-condensés, l'atome d'azote étant commun aux deux cycles ;

• les trois radicaux $R_1$ à $R_3$ pouvant en outre définir avec l'atome d'azote un hétérocycle comportant une double liaison carbone-azote, comportant le cas échéant un double liaison carbone-carbone éventuellement commune à l'hétérocycle et à un noyau benzénique.

– $R_4$, $R_5$, $R_6$ et $R_7$ sont identiques ou différents et représentent :

• un atome d'hydrogène ;

• un radical alkyle linéaire ou ramifié contenant de 1 à 4 atomes de carbone ;

• deux des radicaux $R_4$ à $R_7$, portés par des atomes de carbones adjacents du cycle, pouvant former ensemble un radical akylène, alcénylène ou alcadiènylène, contenant 4 atomes de carbone ou un groupement formé de deux radicaux alkylènes, identiques ou différents, comportant de 1 à 3 atomes de carbone et reliés entre eux par un atome d'oxygène ou de soufre.

8. Procédé selon la revendication 7, caractérisé en ce que l'amine tertiaire répond à la formule (II) dans laquelle l'un au moins des radicaux $R_1$ à $R_3$ représente un radical alkyle ou alcényle linéaire non substitué.

9. Procédé selon la revendication 7, caractérisé en ce que l'amine tertiaire répond à la formule (II) dans laquelle l'un au moins des radicaux $R_1$ à $R_3$ représente un radical aryle, les deux autres, identiques ou différents, représentent des radicaux alkyles linéaires et non substitués.

10. Procédé selon la revendication 7, caractérisé en ce que l'amine tertiaire répond à la formule (II) dans laquelle les radicaux $R_1$ à $R_3$ sont identiques et choisis parmi les radicaux alkyles linéaires contenant au maximum 4 atomes de carbone.

11. Procédé selon la revendication 10, caractérisé en ce que l'amine utilisée est la triéthylamine.

12. Procédé selon la revendication 7, caractérisé en ce que l'amine tertiaire répond à la formule (III) dans laquelle au moins deux des radicaux $R_4$ à $R_7$ représentent l'hydrogène et les deux autres radicaux identiques ou différents représentent un radical alkyle linéaire comportant au maximum 4 atomes de carbone, ces deux radicaux lorsqu'ils sont portés par des atomes de carbone adjacents du cycle pouvant former ensemble un radical alkylène, alcénylène ou alcadiènylène contenant 4 atomes de carbone ou un groupement formé de deux radicaux éthylène reliés entre eux par un atome d'oxygène.

13. Procédé selon la revendication 12, caractérisé en ce que l'amine utilisée est la pyridine.

14. Procédé selon l'une quelconque des revendications précédentes, caractérisé en ce que le palladium représente 0,01 à 10 moles pour 100 moles de chlorobutène éventuellement quaternisé.

15. Procédé selon la revendication 14, caractérisé en ce que le palladium représente de 0,5 à 5 moles pour 100 moles de chlorobutène éventuellement quaternisé.

16. Procédé selon l'une quelconque des revendications précédentes, caractérisé en ce que le catalyseur au palladium est choisi dans le groupe comprenant le dichlorure de palladium et le bis(dibenzylidèneacétone)palladium.

17. Procédé selon l'une quelconque des revendications précédentes, caractérisé en ce que l'alcool répond à la formule R OH dans laquelle R représente un radical alkyle comportant au maximum 12 atomes de carbone éventuellement substitué par un ou deux groupes hydroxyles, un radical cycloalkyle ayant de 5 à 7 atomes de carbone, un radical aralkyle ayant de 7 à 12 atomes de carbone ou un radical phényle.

18. Procédé selon la revendication 17, caractérisé en ce que R représente un radical alkyle comportant au maximum 4 atomes de carbone.

19. Procédé selon l'une quelconque des revendications précédentes, caractérisé en ce que le rapport molaire alcool/chlorobutène éventuellement quaternisé est compris entre 0,5 et 10 et, de préférence entre 0,8 et 5.

20. Procédé selon l'une quelconque des revendications précédentes, caractérisé en ce que la température de l'"alcoxycarbonylation" est comprise entre 60°C et 160°C et, de préférence entre 70°C et 110°C.

21. Procédé selon l'une quelconque des revendications précédentes, caractérisé en ce que la pression de l'"alcoxycarbonylation" est supérieure à 50 bar et, de préférence comprise entre 80 et 200 bar.

## Ansprüche

1. Verfahren zur Herstellung von Estern der Hexen-1,6-disäure durch Umsetzung von Kohlenmonoxid, eines Alkohols und mindestens eines Dichlorbutens in Gegenwart einer katalytisch wirksamen Menge Palladium oder einer Palladiumverbindung, wobei die Umsetzung durch ein tertiäres Amin unterstützt wird, dessen Menge 2 Äquivalente, bezogen auf Dichlorbuten, nicht übersteigt.

2. Verfahren zur Herstellung von Estern der Hexen-1,6-disäure durch Umsetzung von Kohlenmonoxid, eines Alkohols und mindestens eines quaternären Ammoniumchlorids, wobei das Chlorid aus der Quaternisierung eines tertiären Amins mit einem Dichlorbuten erhalten worden ist, in Gegenwart einer katalytisch wirksamen Menge Palladium oder einer Palladiumverbindung.

3. Verfahren zur Herstellung von Estern der Hexen-1,6-disäure, umfassend in einer ersten Stufe die Herstellung eines quaternären Ammoniumchlorids durch Quaternisierung eines tertiären Amins mit einem Dichlorbuten, daran anschließend, wenn erforderlich, die Abtrennung des quaternären Ammoniumchlorids aus dem Reaktionsmedium, und in einer zweiten Stufe das Umsetzen dieses Chlorids mit Kohlenmonoxid und einem Alkohol in Gegenwart einer katalytisch wirksamen Menge Palladium oder einer Palladiumverbindung und gegebenenfalls eines tertiären Amins, dessen in den beiden Stufen eingesetzte Gesamtmenge 2 Äquivalente, bezogen auf Dichlorbuten, nicht übersteigt.

4. Verfahren nach einem der vorangehenden Ansprüche, dadurch gekennzeichnet, daß das tertiäre Amin in einer Menge von 1 Äquivalent ± 20%, bezogen auf Dichlorbuten, eingesetzt wird.

5. Verfahren nach einem der vorangehenden Ansprüche, dadurch gekennzeichnet, daß das tertiäre Amin in einer Menge von 1 Äquivalent ± 10%, bezogen auf Dichlorbuten, eingesetzt wird

6. Verfahren nach einem der vorangehenden Ansprüche, dadurch gekennzeichnet, daß die Umsetzung von Kohlenmonoxid einem Alkohol und Dichlorbuten, gegebenenfalls quaternisiert, in flüssiger Phase durchgeführt wird.

7. Verfahren nach einem der vorangehenden Ansprüche, dadurch gekennzeichnet, daß das tertiäre Amin einer der folgenden Formeln (II) und (III) entspricht :

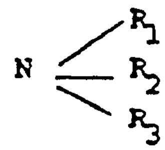

(II)

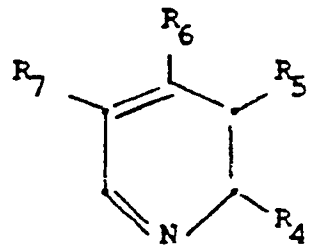

(III)

in denen

– $R_1$, $R_2$ und $R_3$ gleich oder verschieden sind und jeweils stehen für:

• eine lineare oder verzweigte Alkylgruppe mit 1 bis 16 Kohlenstoffatomen, gegebenenfalls substituiert durch eine Phenylgruppe;

• eine lineare oder verzweigte Alkenylgruppe mit 2 bis 12 Kohlenstoffatomen, vorzugsweise mit 4 bis 8 Kohlenstoffatomen;

• eine Arylgruppe mit 6 bis 10 Kohlenstoffatomen, gegebenenfalls substituiert durch eine oder zwei Alkylgruppen mit 1 bis 4 Kohlenstoffatomen;

• wobei zwei der Gruppen $R_1$ bis $R_3$ zusammen eine lineare oder verzweigte Alkylen-, Alkenylen- oder Alkadienylengruppe mit 3 bis 6 Kohlenstoffatomen oder eine Gruppe bilden können, die aus zwei Alkylengruppen gebildet ist, die gleich oder verschieden sind, und 1 bis 3 Kohlenstoffatome enthalten und miteinander durch ein Sauerstoff- oder Schwefelatom verbunden sind;

• und wobei die drei Gruppen $R_1$ bis $R_3$ miteinander verbunden sein können, um mit dem Stickstoffatom eine Verbindung mit zwei ortho-kondensierten Ringen zu bilden, wobei das Stickstoffatom beiden Ringen angehört;

• oder wobei die drei Gruppen $R_1$ bis $R_3$ außerdem mit dem Stickstoffatom einen Heterocyclus bilden können, der eine Kohlenstoff-Stickstoffdoppelbindung enthält, und gegebenenfalls eine Kohlenstoff-Kohlenstoffbindung enthält, die gegebenenfalls dem Heterocyclus und einem Benzolring gemeinsam ist;

– $R_4$, $R_5$, $R_6$ und $R_7$ gleich oder verschieden sein können und bedeuten:

• ein Wasserstoffatom;

• eine lineare oder verzweigte Alkylgruppe mit 1 bis 4 Kohlenstoffatomen;

• wobei zwei der Gruppen $R_4$ bis $R_7$ an benachbarten Kohlenstoffatomen des Rings zusammen eine Alkylen-, Alkenylen-oder Alkadienylengruppe bilden können, die 4 Kohlenstoffatome enthält, oder eine Gruppe, die aus zwei Alkylengruppen gebildet ist, die gleich oder verschieden sind, 1 bis 3 Kohlenstoffatome enthalten und miteinander durch ein Sauerstoff- oder Schwefelatom verbunden sind.

8. Verfahren nach Anspruch 7, dadurch gekennzeichnet, daß das tertiäre Amin der Formel (II) entspricht, worin mindestens einer der Substituenten $R_1$ und $R_3$ eine lineare, nicht-substituierte Alkyl- oder Alkenylgruppe bedeutet.

9. Verfahren nach Anspruch 7, dadurch gekennzeichnet, daß das tertiäre Amin der Formel (II) entspricht, worin mindestens einer der Substituenten $R_1$ bis $R_3$ für eine Arylgruppe steht und die beiden anderen, gleich oder verschieden, lineare und nichtsubstituierte Alkylgruppen bedeuten.

10. Verfahren nach Anspruch 7, dadurch gekennzeichnet, daß das tertiäre Amin der Formel (II) entspricht, in der die Substituenten $R_1$ bis $R_3$ gleich und unter den linearen Alkylgruppen, die maximal 4 Kohlenstoffatome enthalten, ausgewählt sind.

11. Verfahren nach Anspruch 10, dadurch gekennzeichnet, daß das eingesetzte Amin Triethylamin ist.

12. Verfahren nach Anspruch 7, dadurch gekennzeichnet, daß das tertiäre Amin der Formel (III) entspricht, worin mindestens zwei der Substituenten $R_4$ bis $R_7$ für Wasserstoff stehen und die beiden anderen

Substituenten gleich oder verschieden sind und eine lineare Alkylgruppe mit maximal 4 Kohlenstoffatomen bedeuten, wobei diese beiden Gruppen, wenn sie sich an benachbarten Kohlenstoffatomen des Ringes befinden, zusammen eine Alkylen-, Alkenylen- oder Alkadienylengruppe mit 4 Kohlenstoffatomen oder eine Gruppe aus zwei Ethylengruppen, die untereinander durch ein Sauerstoffatom verbunden sind, bilden können.

13. Verfahren nach Anspruch 12, dadurch gekennzeichnet, daß das eingesetzte Amin Pyridin ist.

14. Verfahren nach einem der vorangehenden Ansprüche, dadurch gekennzeichnet, daß das Palladium 0,01 bis 10 mol auf 100 mol Chlorbuten, gegebenenfalls quaternisiert, ausmacht.

15. Verfahren nach Anspruch 15, dadurch gekennzeichnet, daß das Palladium 0,5 bis 5 mol auf 100 mol Chlorbuten, gegebenenfalls quaternisiert, ausmacht.

16. Verfahren nach einem der vorangehenden Ansprüche, dadurch gekennzeichnet, daß der Palladiumkatalysator aus der Gruppe ausgewählt wird, die Palladiumdichlorid und Bis(dibenzylidenaceton)palladium umfaßt.

17. Verfahren nach einem der vorangehenden Ansprüche, dadurch gekennzeichnet, daß der Alkohol der Formel ROH entspricht, in der R eine Alkylgruppe mit maximal 12 Kohlenstoffatomen, gegebenenfalls substituiert mit einer oder zwei Hydroxylgruppen, eine Cycloalkylgruppe mit 5 bis 7 Kohlenstoffatomen, eine Aralkylgruppe mit 7 bis 12 Kohlenstoffatomen oder eine Phenylgruppe bedeutet.

18. Verfahren nach Anspruch 17, dadurch gekennzeichnet, daß R für eine Alkylgruppe mit maximal 4 Kohlenstoffatomen steht.

19. Verfahren nach einem der vorangehenden Ansprüche, dadurch gekennzeichnet, daß das Molverhältnis Alkohol/Chlorbuten, gegebenenfalls quaternisiert, 0,5 bis 10, vorzugsweise 0,8 bis 5, ausmacht.

20. Verfahren nach einem der vorangehenden Ansprüche, dadurch gekennzeichnet, daß die Temperatur der "Alkoxycarbonylierung" zwischen 60 und 160°C, vorzugsweise zwischen 70 und 110°C, liegt.

21. Verfahren nach einem der vorangehenden Ansprüche, dadurch gekennzeichnet, daß der Druck der "Alkoxycarbonylierung" über 50 bar, vorzugsweise zwischen und 80 und 200 bar, beträgt.

## Claims

1. Process for the preparation of hexene-1,6-dioates by reacting carbon monoxide, an alcohol and at least one dichlorobutene in the presence of a catalytically effective quantity of palladium or of a palladium compound, the reaction being assisted by a tertiary amine, the quantity of which does not exceed 2 equivalents relative to the dichlorobutene.

2. Process for the preparation of hexene-1,6-dioates by reacting carbon monoxide, an alcohol and at least one quaternary ammonium chloride, the said chloride resulting from the quaternization of a tertiary amine by a dichlorobutene, in the presence of a catalytically effective quantity of palladium or of a palladium compound.

3. Process for the preparation of hexene-1,6-dioates, which consists, in a first stage, in preparing a quaternary ammonium chloride by quaternization of a tertiary amine using a dichlorobutene, followed, where appropriate, by the separation of the said quaternary ammonium chloride from the reaction medium, and in a second stage reacting the said chloride with carbon monoxide and an alcohol in the presence of a catalytically effective quantity of palladium or of a palladium compound and, where appropriate, of a tertiary amine, or which the total quantity employed in the two stages does not exceed 2 equivalents relative to the dichlorobutene.

4. Process according to any one of the preceding claims, characterized in that the tertiary amine is employed at a rate of 1 equivalent, within plus or minus 20%, relative to the dichlorobutene.

5. Process according to any one of the preceding claims, characterized in that the tertiary amine is employed at a rate of 1 equivalent, within plus or minus 10%, relative to the dichlorobutene.

6. Process according to any one of the preceding claims, characterized in that the reaction between carbon monoxide, an alcohol and dichlorobutene which is quaternized where appropriate, is carried out in a liquid phase.

7. Process according to any one of the preceding claims, characterized in that the tertiary amine corresponds to any one of the formulae (II) and (III) below.

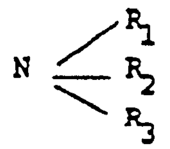

(II)

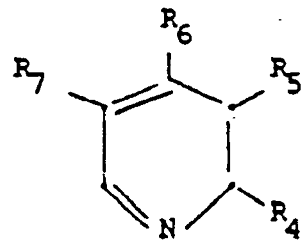

(III)

in which :

$R_1$, $R_2$ and $R_3$ are identical or different and represent :

a straight-chain or branched alkyl radical containing from 1 to 16 carbon atoms, optionally substituted with a phenyl group ;

a straight-chain or branched alkenyl radical containing from 2 to 12 carbon atoms, preferably from 4 to 8 carbon atoms ;

an aryl radical containing from 6 to 10 carbon atoms, optionally substituted with one or two alkyl radicals containing from 1 to 4 carbon atoms ;

it being possible for two of the said radicals $R_1$ to $R_3$ to together form a straight-chain or branched alkylene, alkenylene or alkadienylene radical containing from 3 to 6 carbon atoms or a group formed from two alkylene radicals, which can be identical or different, containing from 1 to 3 carbon atoms and linked to each other through an oxygen or sulfur atom ;

it being possible for the three radicals $R_1$ to $R_3$ to be linked to one another to form, with the nitrogen atom, a compound containing two ortho-fused rings, the nitrogen atom being common to the two rings ; and

it being additionally possible for the three radicals $R_1$ to $R_3$ to define, with the nitrogen atom, a heterocycle containing a carbonnitrogen double bond, containing, where appropriate, a carbon-carbon double bond optionally common to the heterocycle and to a benzene ring.

$R_4$, $R_5$, $R_6$ and $R_7$ are identical or different and represent :

a hydrogen atom ;

a straight-chain or branched alkyl radical containing from 1 to 4 carbon atoms ; and

it being possible for two of the radicals $R_4$ to $R_7$, carried by the adjacent carbon atoms of the ring, to together form an alkylene, alkenylene or alkadienylene radical containing four carbon atoms or a group formed from two alkylene radicals, which can be identical or different, containing from 1 to 3 carbon atoms and linked to each other through an oxygen or sulfur atom.

8. Process according to claim 7, characterized in that the tertiary amine corresponds to formula (II) in which at least one of the radicals $R_1$ to $R_3$ represents an unsubstituted straight-chain alkyl or alkenyl radical.

9. Process according to claim 7, characterized in that the tertiary amine corresponds to formula (II) in which at least one of the radicals $R_1$ to $R_3$ represents an aryl radical, and the other two, which may be identical or different, represent alkyl radicals which are straight-chain and unsubstituted.

10. Process according to claim 7, characterized in that the tertiary amine corresponds to formula (II) in which the radicals $R_1$ to $R_3$ are identical and chosen from amongst straight-chain alkyl radicals containing a maximum of 4 carbon atoms.

11. Process according to claim 10, characterized in that the amine employed is triethylamine.

12. Process according to claim 7, characterized in that the tertiary amine corresponds to formula (III) in which at least two of the radicals $R_4$ and $R_7$ represent hydrogen and the other two radicals, which can be identical or different, represent a straightchain alkyl radical containing a maximum of 4 carbon atoms, it being possible for these two radicals, when they are carried by adjacent carbon atoms of the ring, to together form an alkylene, alkenylene or alkadienylene radical containing 4 carbon atoms or a group formed from

two ethylene radicals linked to each other through an oxygen atom.

13. Process according to claim 12, characterized in that the amine employed is pyridine.

14. Process according to any one of the preceding claims, characterized in that the palladium represents 0.01 to 10 moles per 100 moles of optionally quaternized chlorobutene.

15. Process according to claim 14, characterized in that the palladium represents from 0.5 to 5 moles per 100 moles of optionally quaternized chlorobutene.

16. Process according to any one of the preceding claims, characterized in that the palladium catalyst is chosen from the group comprising palladium dichloride and bis(dibenzylideneacetone)palladium.

17. Process according to any one of the preceding claims, characterized in that the alcohol corresponds to the formula ROH in which R represents an alkyl radical containing a maximum of 12 carbon atoms, optionally substituted with one or two hydroxyl groups, a cycloalkyl radical containing from 5 to 7 carbon atoms, an aralkyl radical containing from 7 to 12 carbon atoms or a phenyl radical.

18. Process according to claim 17, characterized in that R represents an alkyl radical containing a maximum of 4 carbon atoms.

19. Process according to any one of the preceding claims, characterized in that the molar ratio alcohol: optionally quaternized chlorobutene is between 0.5 : 1 and 10 : 1 and preferably between 0.8 : 1 and 5 : 1.

20. Process according to any one of the preceding claims, characterized in that the "alkoxycarbonylation" temperature is between 60°C and 160°C and preferably between 70°C and 110°C.

21. Process according to any one of the preceding claims, characterized in that the "alkoxycarbonylation" pressure is higher than 50 bar and preferably between 80 and 200 bar.